# EUROPEAN PATENT SPECIFICATION

(11) **EP 4 412 609 B1**
(45) Date of publication and mention of the grant of the patent: **27.05.2026**
(21) Application number: 22878063.1
(22) Date of filing: 07.10.2022
(51) Int. Cl.: A61K 31/352, C07C 41/03, B01J 27/26, C07F 15/06, C08G 65/26, C07C 51/367, C07D 307/91, C07D 311/58, C07D 311/80

(54) **ALKOXYLATION OF CANNABIDIOL & OTHER CANNABINOIDS**
ALKOXYLIERUNG VON CANNABIDIOL UND ANDEREN CANNABINOIDEN
ALCOXYLATION DE CANNABIDIOL ET D'AUTRES CANNABINOÏDES

(30) Priority: 07.10.2021 US 202163253193 P
(43) Date of publication of application: 14.08.2024
(73) Proprietor: Indorama Ventures Oxides LLC, The Woodlands, TX 77380 (US)
(72) Inventor: LAHASKY, Samuel, Hattiesburg, Mississippi 39402 (US); SHARP, Kip Douglas, The Woodlands, Texas 77381 (US); SLIKTA, Alberto, The Woodlands, Texas 77381 (US); PRESTRIDGE, Britney Hebert, The Woodlands, Texas 77381 (US); BARKER, Emily Mattews, Hattiesburg, Mississippi 39402 (US); RICHARDSON, Ryan, The Woodlands, Texas 77381 (US)
(74) Representative: Weidner Stern Jeschke
(86) International application number: PCT/IB2022/059624
(87) International publication number: WO 2023/057982

(56) References cited:
- EP-A1- 1 927 611
- WO-A1-2008/107879
- WO-A1-2018/091551
- WO-A1-2021/171209
- WO-A2-2012/071149
- US-A1- 2008 132 728
- US-A1- 2015 197 484
- US-A1- 2019 345 087

## Description

### FIELD

A process for the alkoxylation of cannabidiol (CBD) and other cannabinoids using a double metal cyanide (DMC) catalyst is disclosed. This alkoxylation process, which circumvents issues associated with traditional alkoxylation catalysts (e.g., alkaline, or acidic catalysts), is herein described, demonstrating that the use of the DMC catalyst is critical towards the preparation of the alkoxylated cannabinoid compound.

### BACKGROUND

Cannabinoids are gaining popularity in clinical trials due to their effect on the endocannabinoid system, which plays vital roles in regulating a range of bodily functions and processes, including, sleep, appetite, emotion-regulation, and memory. Just a few of the cannabinoid receptors that have been characterized include the G protein-coupled receptors, CB1 and CB2. These two receptors are critical to the endocannabinoid system, as they have been found to occur within and outside the central nervous system, aiding in cannabinoid-induced immunosuppression, the regulation of neurotransmitters and possible anti-inflammatory effects that have been linked with the function of the immune system. Located on the periphery of a cell, these high-affinity cannabinoid receptors have specific binding sites for cannabinoid compounds, and once bound, these cannabinoid agonists induce the physiological responses listed above and potentially more.

Of these cannabinoid receptor agonists, cannabidiol (CBD) has been often studied due to its abundance and non-psychoactive effect on humans. CBD is typically the largest fraction of the cannabinoid-array that can be extracted from the *cannabis* plant. CBD is a phytocannabinoid that consists of the structure ((2-[1R-3-methyl 6-(1 methylethenyl]-2-cyclohexen-1-yl]-5-pentyl-1,3-benzenediol)), and plays a critical, yet complex role in the human body's endocannabinoid system, which is involved in maintaining homeostasis throughout the body.

The use of a purified CBD solution demonstrated dramatic reductions in the number of seizures (as compared to a placebo), illustrating its potential usage as a naturally derived medicinal option for patients with Dravet and Lennox-Gastaut syndromes. Other medicinal uses of CBD range in treatments for chronic pain to addiction, making this one of the most versatile naturally-derived medicinal compounds to be studied.

Due to the clinical significance of the CBD and other cannabinoids, a strong importance has been placed on increasing all cannabinoid's water-solubility, which ultimately affects its bioavailability for the endocannabinoid system. The water solubility of these lipophilic cannabinoid compounds has been studied, demonstrating very little solubility in water (2-10 ug/mL). The hydrophobicity and lipophilic nature of cannabinoids is commonly attributed to their lack of bioavailability, ultimately limiting their potential therapeutic effect as a drug. For these reasons, it would be advantageous to increase the solubility of CBD, and other cannabinoid species, in water through means of (i) chemical reaction (e.g., alkoxylation) and/or (ii) emulsification using the chemically altered cannabinoids as a water-solubility enhancing agent.

Alkoxylation, in particular ethoxylation, is a common method to increase the water-solubility of hydrophobic, fatty-molecules. For instance, fatty-alcoholic or nonyl-phenolic ethoxylates are commonly prepared in large commercial quantities via polymerization epoxides, including ethylene oxide (EO), propylene oxide (PO) and butylene oxide (BO), in the presence of a catalyzed initiator. Typical initiators contain an active free hydrogen, such as a primary or secondary hydroxyl functional group or a phenolic group, which can be catalyzed using an alkaline catalyst. Common alkaline catalysts, including potassium hydroxide and sodium hydroxide, will deprotonate this active hydrogen, resulting in a highly active nucleophilic initiating species. Once the ethylene oxide is added into the reactor, this active nucleophile will undergo ring opening polymerization of the epoxide monomer to give the resulting alcohol- or phenol-ethoxylate. For instance, increasing the molar ratio of epoxide-to-initiator (for instance ethylene oxide and nonylphenol) increases the resulting product's water solubility, and once the threshold of this ratio is determined (10:1 in the case of nonylphenol), and overcome, a hydrophobic compound can be altered into a water-soluble molecule.

While alkoxylation via alkaline catalysis may be a common method to increase the water-solubility of hydrophobic compounds like nonylphenol, this synthetic method cannot be used to alkoxylate CBD and other cannabinoids, as alkaline catalysts are well known to convert the phenolic hydroxyl group in CBD into its quinone form. Both CN108426863B and WO2015158381A1 demonstrate that the cannabidiol quinone derivative is prepared by exposing CBD to a catalyst in the form of a hydroxide of a monovalent cation (e.g., NaOH or KOH). The formation of the quinone derivative could not allow CBD to proceed with the necessary ethoxylation reaction, which would increase its water-solubility. US 2019/345087 A1 discloses a process for the alkoxylation of cannabidiol derivatives.

While alkaline catalysis is the most commonly method used to prepare ethoxylates of fatty compounds, other methods also exist, most notably, acid-catalysis. For instance, the acid catalyzed ethoxylation of fatty alcohols can also result in an ethoxylated alcohol product. A common industrial acid-catalysis is boron trifluoride, which has been known to make narrow-range alcohol ethoxylates. Narrow-range ethoxylates are commonly classified by low quantities of (i) the unreacted alcohol and (ii) high mole ethoxymers. When compared to broad-range ethoxylates with the same molar ratio of epoxide-to-fatty alcohol, narrow range ethoxylates are commonly more soluble in water due to the combination of the low amounts of (i) unreacted alcohol and (ii) high mole ethoxymers.

Unfortunately, while the preparation of a narrow-range alkoxylate could benefit the water-solubility of CBD, the use of boron trifluoride (and its etherates) do not permit the alkoxylation of CBD, as these Lewis acid catalysts are well known to convert CBD into other phytocannabinoids, most notably, tetrahydrocannabinol (THC). The conversion of CBD into THC not only increases the psychoactivity of the phytocannabinoid (as THC is well-known to be much more psychoactive due to its agonistic effect to the CB1 receptor), but it inhibits the ethoxylation of CBD, thereby obstructing the chemical process to increase CBD's water solubility.

Since the alkoxylation of CBD cannot take place under the traditional synthetic procedures, current methods to increase the water solubility of CBD are narrowed to the use of formulating aids that emulsify or solubilize all cannabinoids. While this is common practice, these formulating aids are limited in their ability to increase the loading of cannabinoid molecules into a given formulation/recipe, or they require the use of co-solvents (e.g., ethanol) to solubilize the cannabinoid in the aqueous media (typically in the form of tinctures).

Therefore, it would be highly desirable to provide a new process for alkoxylating cannabinoids in good yield without suffering from the problems associated with the state of the art processes described above.

### SUMMARY

The present disclosure demonstrates (i) the methodology and feasibility of an alkoxylation process of cannabidiol and other cannabinoids in the presence of a double metal cyanide (DMC) catalyst, while also illustrating (ii) the enhanced water solubility of the resulting alkoxylated cannabinoid.

The use of a double metal cyanide (DMC) catalyst avoids the problems that arise with using either alkaline or acid catalysis as described above. Using DMC as the catalyst ensures that the correct conditions are met in order for the cannabinoid to undergo alkoxylation and limits the potential for the occurrence of other side reactions (e.g., quinone-derivatization or the formation of tetrahydrocannabinoid, THC). Being known to behave as a Lewis acid, it is somewhat surprising that DMC can provide the correct conditions for the alkoxylation of the cannabinoid, as boron trifluoride etherate (also a Lewis acid) favors the occurrence of such side reactions. Through DMC's coordination and insertion mechanisms, the DMC catalyst can alkoxylate cannabinoids at relatively mild conditions, resulting in useful alkoxylated molecules. Disclosed is the process in which CBD, and other cannabinoids, are converted into the alkoxylate via the DMC-catalyzed alkoxylation of these cannabinoid hydrophobes. As expected with all ethoxylated fatty compounds, the alkoxylated cannabinoid has increased water solubility as compared to the non-alkoxylated cannabinoid.

### BRIEF DESCRIPTION OF THE DRAWINGS

Figure 1 depicts the FTIR of CBD and e-CBD;
Figure 2 depicts the ¹H NMR's of CBD and e-CBD in CDCl₃; and

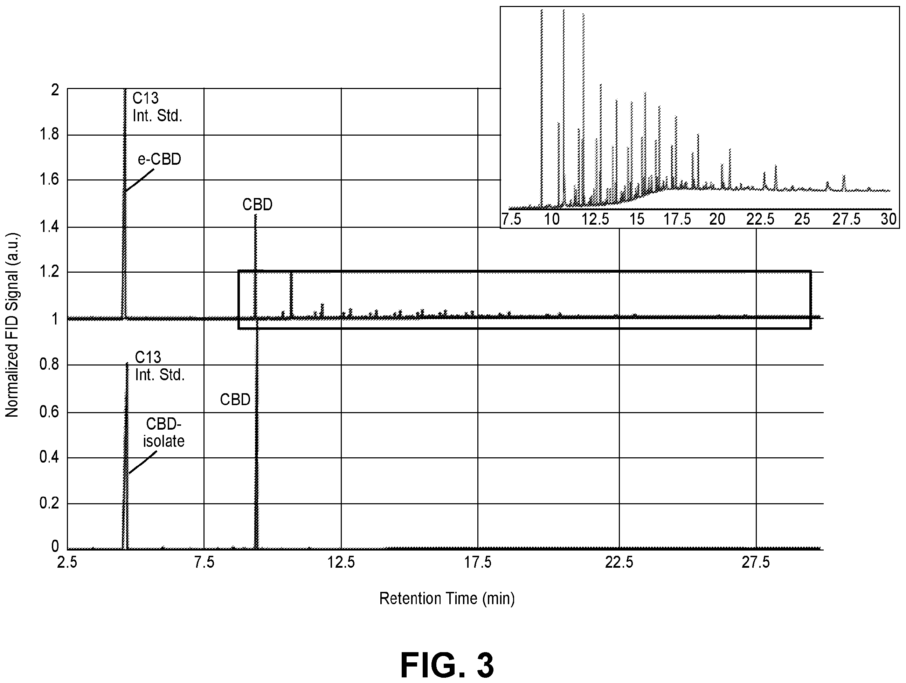

Figure 3 depicts the GC-FID chromatograms of the CBD-isolate and e-CBD.

### DETAILED DESCRIPTION

The subject matter of the present disclosure is generally directed to a process and methodology in which the correct conditions are met in order to produce alkoxylated cannabinoid hydrophobic molecules, such as alkoxylated CBD, using a double metal cyanide (DMC) catalyst. The process comprises (i) forming a mixture of a cannabinoid, an alkylene oxide and a double metal cyanide (DMC) catalyst, the DMC catalyst being represented by the formula Mb[M1 (CN)r(X)t]c[M2(X)6]d. n M3yAy
where M and M³ are each metals; M¹ is a transition metal different from M, each X represents a group other than cyanide that coordinates with the M¹ ion; M² is a transition metal; A represents an anion; b, c and d are numbers that reflect an electrostatically neutral complex; r is from 4 to 6; t is from 0 to 2; x and y are integers that balance the charges in the metal salt M³ ₓ A_{y} , and n is zero or a positive integer, and (ii) subjecting the mixture to conditions sufficient to activate the DMC catalyst and to alkoxylate the cannabinoid to form the alkoxylated cannabinoid.

The following terms shall have the following meanings:
The term "comprising" and derivatives thereof are not intended to exclude the presence of any additional component, step, or procedure, whether or not the same is disclosed herein. To avoid any doubt, all compositions claimed herein through use of the term "comprising" may include any additional additive or compound, unless stated to the contrary. In contrast, the term, "consisting essentially of" if appearing herein, excludes from the scope of any succeeding recitation any other component, step, or procedure, except those that are not essential to operability and the term "consisting of", if used, excludes any component, step or procedure not specifically delineated or listed. The term "or", unless stated otherwise, refers to the listed members individually as well as in any combination.

The articles "a" and "an" are used herein to refer to one or to more than one (i.e., to at least one) of the grammatical objects of the article. By way of example, "a cannabinoid" means one cannabinoid or more than one cannabinoid. The phrases "in one embodiment", "according to one embodiment" and the like generally mean the particular feature, structure, or characteristic following the phrase is included in at least one embodiment of the present disclosure and may be included in more than one embodiment of the present disclosure. Importantly, such phrases do not necessarily refer to the same aspect. If the specification states a component or feature "may", "can", "could", or "might" be included or have a characteristic, that particular component or feature is not required to be included or have the characteristic.

The term "about" as used herein can allow for a degree of variability in a value or range, for example, it may be within 10%, within 5%, or within 1% of a stated value or of a stated limit of a range.

Values expressed in a range format should be interpreted in a flexible manner to include not only the numerical values explicitly recited as the limits of the range, but to also include all of the individual numerical values or sub-ranges encompassed within that range as if each numerical value and sub-range is explicitly recited. For example, a range such as from 1 to 6, should be considered to have specifically disclosed sub-ranges, such as, from 1 to 3, from 2 to 4, from 3 to 6, etc., as well as individual numbers within that range, for example, 1, 2, 3, 4, 5, and 6. This applies regardless of the breadth of the range.

The terms "preferred" and "preferably" refer to embodiments that may afford certain benefits, under certain circumstances. However, other embodiments may also be preferred, under the same or other circumstances. Furthermore, the recitation of one or more preferred embodiments does not imply that other embodiments are not useful and is not intended to exclude other embodiments from the scope of the present disclosure.

As used herein, the term "cannabinoid" refers to a compound from a class of molecules commonly found in plants of the genus cannabis and their derivatives (i.e., a chemical compound having a structure similar to a cannabinoid but which includes at least one functional group that does not occur in nature). The cannabinoid may be an oily extract of a cannabis type plant, or pure cannabinoid or synthetic or cannabinoid derivative. Many cannabinoids can be identified by the "cannabi" text in their chemical name. There are at least 113 different cannabinoids isolated from cannabis, exhibiting varied (similar and different) effects.

Examples of cannabinoids within the context of this disclosure include the following molecules: Cannabichromene (CBC), Cannabichromenic acid (CBCA), Cannabichromevarin (CBCV), Cannabichromevarinic acid (CBCVA), Cannabicyclol (CBL), Cannabicyclolic acid (CBLA), Cannabicyclovarin (CBLV), Cannabidiol (CBD), Cannabidiol monomethylether (CBDM), Cannabidiolic acid (CBDA), Cannabidiorcol (CBD-C1), Cannabidivarin (CBDV), Cannabidivarinic acid (CBDVA), Cannabielsoic acid B (CBEA-B), Cannabielsoin (CBE), Cannabielsoin acid A (CBEA-A), Cannabigerol (CBG), Cannabigerol monomethylether (CBGM), Cannabigerolic acid (CBGA), Cannabigerolic acid monomethylether (CBGAM), Cannabigerovarin (CBGV), Cannabigerovarinic acid (CBGVA), Cannabinodiol (CBND), Cannabinodivarin (CBDV), Cannabinol (CBN), Cannabinol methylether (CBNM), Cannabinol-C2 (CBN-C2), Cannabinol-C4 (CBN-C4), Cannabinolic acid (CBNA), Cannabiorcool (CBN-C1), Cannabivarin (CBV), Cannabitriol (CBT), Cannabitriolvarin (CBTV), 10-Ethoxy-9-hydroxy-delta-6a-tetrahydrocannabinol, Cannbicitran (CBT), Cannabiripsol (CBR), 8,9-Dihydroxy-delta-6a-tetrahydrocannabinol, Delta-8-tetrahydrocannabinol (Δ8-THC), Delta-8-tetrahydrocannabinolic acid (Δ8-THCA), Delta-9-tetrahydrocannabinol (THC), Delta-9-tetrahydrocannabinol-C4 (THC-C4), Delta-9-tetrahydrocannabinolic acid A (THCA-A), Delta-9-tetrahydrocannabinolic acid B (THCA-B), Delta-9-tetrahydrocannabinolic acid-C4 (THCA-C4), Delta-9-tetrahydrocannabiorcol (THC-C1), Delta-9-tetrahydrocannabiorcolic acid (THCA-C1), Delta-9-tetrahydrocannabivarin (THCV), Delta-9-tetrahydrocannabivarinic acid (THCVA), 10-Oxo-delta-6a-tetrahydrocannabinol (OTHC), Cannabichromanon (CBCF), Cannabifuran (CBF), Cannabiglendol, Delta-9-cis-tetrahydrocannabinol (cis-THC), Tryhydroxy-delta-9-tetrahydrocannabinol (triOH-THC), Dehydrocannabifuran (DCBF), and 3,4,5,6-Tetrahydro-7-hydroxy-alpha-alpha-2-trimethyl-9-n-propyl-2,6-methano-2H-1-benzoxocin-5-methanol.

Cannabinoid derivatives include, but are not limited to, 11-hydroxy derivatives, 3-(1',1'-dimethylheptyl) derivatives, 9-substituted derivatives, 1'-substituted derivatives, propyl analogues, deoxy derivatives, and prodrug ester derivatives, for example of Δ⁹-THC and Δ⁸-THC. Other derivatives include cannabinoid analogues with aliphatic side chains, such as heptynyl, heptenyl, octynyl, octenyl, bromohexynyl, bromohexenyl, nonynyl, and other side chains with double or triple bonds.

As used herein, term "cannabidiol" or "CBD" will refer to the isolate form of CBD, which exists as a powder, and in some embodiments is greater than 98% pure.

As with most plant derived extracts and oils, cannabinoid compounds are known for their limited water-solubility, making their dissolution into aqueous media difficult to achieve. Limits on their weight fractions into an aqueous formulation are constrained by their properties or the formulator's ability to select the proper formulating aids. Some common methods to increase the water solubility of a hydrophobic molecule is to either (i) chemically alter the structure of the molecule or (ii) prepare a formulation in which formulating aids are used. One method to chemically alter a hydrophobic compound is to create the salt of the compound (if possible) or to alkoxylate the oxyalkylatable groups of the compound (if possible). While the formation of the salt of CBD and other cannabinoids may be possible through some means of chemistry, this disclosure focuses on a different method of altering the structure of the cannabinoid molecule by the alkoxylation of CBD or other cannabinoid using an alkylene oxide, such as ethylene oxide (EO), propylene oxide (PO), butylene oxide (BO) or mixtures thereof, in the presence of a double metal cyanide (DMC) catalyst. The resulting product exhibits an increased or enhanced water solubility, and thus in another embodiment the present disclosure provides a method for enhancing the water solubility of a cannabinoid by alkoxylating the cannabinoid in the presence of an alkylene oxide and a DMC catalyst. By "enhanced solubility" it is meant the alkoxylated cannabinoid of the present disclosure has an increased water solubility of at least about 5% or at least about 10% over the water solubility of the non-alkoxylated precursor cannabinoid (i.e., the cannabinoid before alkoxylation). In another embodiment, the term refers to an increase of at least about 20%, or at least about 30%, or at least 40%, or at least about 50%, or at least about 60% or at least about 70% or at least about 80%, or at least about 100% (2-fold) over the water solubility of the non-alkoxylated precursor cannabinoid. In another embodiment, the increase is at least about 3-fold, or at least about 4-fold, at least about 5-fold, or at least about 6-fold, or at least about 8-fold or at least 10-fold over the water solubility of the non-alkoxylated cannabinoid. In another embodiment, the increase is at least 15-fold. Each possibility represents a separate embodiment of the present disclosure.

Alkoxylated products made using the above reaction conditions can vary widely in their properties depending on the predetermined molar ratios of the cannabinoid starter compound and the alkylene oxide. This starter-to-alkylene oxide molar ratio is a common method to determine properties such as melting point, crystallinity, water solubility and cloud point. For the purposes of this disclosure, this molar ratio will be referred to as the degrees of polymerization of the alkylene oxide, or the polyoxy-alkoxide value. For instance, when the molar ratio of ethylene oxide-to-cannabinoid is 10-to-1, respectively, the polyoxyethylene (POE) value will be considered 10 (i.e., (POE 10)). Similarly, POP and POB refer to the polyoxypropylene and polyoxybutylene iterations of this molar ratio, respectively. For the purposes of this disclosure, the POE, POP and POB values are rounded to the nearest whole number for simplicity.

Double metal cyanide (DMC) catalysts may be used to catalyze the reaction between an alkylene oxide and compounds that contain a free active hydrogen (e.g., the hydrogen from the hydroxyl-group within a fatty alcohol). The kinetics of this reaction are surprisingly rapid as compared to the alkoxylation kinetics of an alkaline-catalyzed system (i.e., potassium hydroxide and sodium hydroxide). The amount of DMC catalyst used is generally expressed in terms of parts by weight of metals in the catalyst per million parts by weight of the alkoxylated product. According to one embodiment, the concentration of the DMC catalyst can be from about 5 ppm to about 1000 ppm, or from about 5 ppm to about 500 ppm, or from about 5 ppm to about 100 ppm of the alkoxylated product.

In one embodiment, the DMC catalyst can be represented by the formula

M_{b}[M¹(CN)ᵣ(X)ₜ]_{c}[M²(X)₆]_{d} · nM³ₓA_{y}

where M and M³ are each metals; M¹ is a transition metal different from M, each X represents a group other than cyanide that coordinates with the M¹ ion; M² is a transition metal; A represents an anion; b, c and d are numbers that reflect an electrostatically neutral complex; r is from 4 to 6; t is from 0 to 2; x and y are integers that balance the charges in the metal salt M³ₓA_{y}, and n is zero or a positive integer. The foregoing formula does not reflect the presence of neutral small organic ligand complexing agents, such as t-butanol, which are often present in the DMC catalyst complex.

M and M³ each are preferably a metal ion independently selected from the group consisting of Zn²⁺, Fe²⁺, Co⁺²⁺, Ni²⁺, Mo⁴+, Mo⁶⁺, Al⁺³⁺, V⁴⁺, V⁵⁺, Sr²⁺, W⁴⁺, W⁶⁺, Mn²⁺, Sn²⁺, Sn⁴⁺, Pb²⁺, Cu²⁺, La³⁺ and Cr³⁺, with Zn²⁺ being preferred.

M¹ and M² are preferably Fe³⁺, Fe²⁺, Co³⁺, Co²⁺, Cr²⁺, Cr³⁺, Mn²⁺, Mn³⁺, Ir³⁺, Ni²⁺, Rh³⁺, Ru²⁺, V⁴⁺, V⁵⁺, Ni²⁺, Pd²⁺, and Pt²⁺. Among the foregoing, those in the plus-three oxidation state are more preferred as the M¹ and M² metal. Co³⁺ and Fe³⁺ are even more preferred and Co³⁺ is most preferred.

Suitable anions A include, but are not limited to, halides such as chloride, bromide and iodide, nitrate, sulfate, carbonate, cyanide, oxalate, thiocyanate, isocyanate, perchlorate, isothiocyanate, an alkanesulfonate such as methanesulfonate, an arylenesulfonate such as p-toluenesulfonate, trifluoromethanesulfonate (triflate) and a C₁₋₄ carboxylate. Chloride ion is especially preferred.

r is preferably 4, 5 or 6, preferably 4 or 6, and most preferably 6; t is preferably 0 or 1, most preferably 0. In most cases, r + t will equal six.

The molar ratio of c:d is advantageously from about 100:0 to about 20:80, more preferably from about 100:0 to about 50:50, and even more preferably from about 100:0 to about 80:20.

Specific examples of DMC catalysts which may be used are described, for example in U.S. Patent No. 4,500,704, and may include: zinc hexacyanoferrate (EH), zinc hexacyanoferrate (II), nickel (II) hexacyanoferrate (II), nickel(II) hexacyanoferrate (III), zinc hexacyanoferrate (III) hydrate, cobalt(II) hexacyanoferrate (II), nickel(II) hexacyanoferrate (III) hydrate, ferrous hexacyanoferrate (III), cobalt (II) hexacyanocobaltate (III), zinc hexacyanocobaltate (II), zinc hexacyanomanganate (II), zinc hexacyanochromate (III), zinc iodopentacyanoferrate (III), cobalt (II) chloropentacyanoferrate (II), cobalt (II) bromopentacyanoferrate (II), iron (II) fluoropentacyanoferrate (II), zinc chlorobromotetracyanoferrate (III), iron (III) hexacyanoferrate (III), aluminum dichlorotetracyanoferrate (III), molybdenum (IV) bromopentacyanoferrate (II), molybdenum (VI) chloropentacyanoferrate (II), vanadium (IV) hexacyanochromate (II), vanadium (V) hexacyanoferrate (III), strontium (II) hexacyanomanganate (III), tungsten (IV) hexacyano vanadate (IV), aluminumchloropentacyano vanadate(V), tungsten (VI) hexacyanoferrate (III), manganese (II) hexacyanoferrate (II) and chromium (III) hexacyanoferrate (III). Still other cyanide complexes can also be used such as Zn[Fe(CN)₅NO], Zn₃[Fe(CN)₅NO₂]₂, Zn[Fe (CN)₅CO], Zn(Fe(CN)₅H₂O), Fe[Fe(CN)₅OH], Cr[Fe(CN)₅NCO), Cr[Fe(CN)₅NCS), Al(Co(CN)₅CNO) and Ni₃[Mn(CN)₅CNS]₂. Preferably, the double metal cyanide salt is zinc hexacyanocobaltate.

In some embodiments, the DMC catalyst may be complexed with a small organic ligand as noted above. Organic ligands which may be used include alcohols, aldehydes, ketones, ethers, amides, nitriles, sulfides and mixtures thereof.

Suitable alcohols include monoalcohols and polyalcohols. Examples of monoalcohols include methanol, ethanol, n-propanol, isopropanol, n-butanol, isobutanol, t-butanol, octanol, octadecanol, 3-butyn-1-ol, 3-butene-1-ol, propargyl alcohol, 2-methyl-2-propanol, 2-methyl-3-butyn-2-ol, 2-methyl-3-butene-2-ol, 3-butyn-1-ol, 3-butene-1-ol and 1-t-butoxy-2-propanol. Monoalcohols may also include halogenated alcohols such as 2-chloroethanol, 2-bromoethanol, 2-chloro-1-propanol, 3-chloro-1-propanol, 3-bromo-1-propanol, 1,3-dichloro-2-propanol, 1-chloro-2-methyl-2-propanol as well as nitroalcohols, keto-alcohols, ester-alcohols, cyanoalcohols, and other inertly substituted alcohols.

Examples of polyalcohols include ethylene glycol, propylene glycol, glycerine, 1,1,1-trimethylol propane, 1,1,1-trimethylol ethane, 1,2,3-trihydroxybutane, pentaerythritol, xylitol, arabitol, mannitol, 2,5-dimethyl-3-hexyn-2,5-diol, 2,4,7,9-tetramethyl-5-decyne-4,7-diol, sucrose, sorbitol, alkyl glucosides, such as methyl glucoside and ethyl glucoside. Low molecular weight polyether polyols, particularly those having an equivalent weight of about 350 or less, more preferably about 125-250, are also useful organic ligand. complexing agents.

Suitable aldehydes include formaldehyde, acetaldehyde, butyraldehyde, valeric aldehyde, glyoxal, benzaldehyde and toluic aldehyde. Suitable ketones include acetone, methyl ethyl ketone, 3-pentanone and 2-hexanone.

Suitable ethers include cyclic ethers, such as dioxane, trioxymethylene and paraformaldehyde as well as acyclic ethers, such as diethyl ether, 1-ethoxy pentane, bis(betachloro ethyl) ether, methyl propyl ether, diethoxy methane, dialkyl ethers of alkylene or polyalkylene glycols (such as ethylene glycol dimethyl ether, diethylene glycol dimethyl ether, triethylene glycol dimethyl ether and octaethylene glycol dimethyl ether).

Amides such as formamide, acetamide, propionamide, butyramide and valeramide are useful organic ligand complexing agents. Esters such as amyl formate, ethyl formate, hexyl formate, propyl formate, ethyl acetate, methyl acetate and triethylene glycol diacetate can be used as well. Suitable nitriles include acetonitrile and proprionitrile. Suitable sulfides include dimethyl sulfide, diethyl sulfide, dibutyl sulfide and diamyl sulfide.

Preferred organic ligands are t-butanol, 1-t-butoxy-2-propanol, polyether polyols having an equivalent weight of about 75-350 and dialkyl ethers of alkylene and polyalkylene glycols. Especially preferred complexing agents are t-butanol, 1-t-butoxy-2-propanol, polyether polyols having an equivalent weight of 125-250 and a dimethyl ether of mono-, di- or triethylene glycol.

Preferably, the organic ligand is t-butanol.

According to one embodiment, the alkylene oxide may be, for example, ethylene oxide, 1,2-propylene oxide, 2,3- propylene oxide, 1,2-butane oxide, 2-methyl-1,2-butaneoxide, 2,3-butane oxide, tetrahydrofuran, epichlorohydrin, hexane oxide, styrene oxide, divinylbenzene dioxide, a glycidyl ether such as Bisphenol A diglycidyl ether, or other polymerizable oxirane and mixtures thereof. According to one preferred embodiment the alklyene oxide may be ethylene oxide, 1,2-propylene oxide, 1,2-butylene oxide or a mixture thereof.

In this disclosure, the DMC catalyst is used alongside the cannabinoid as a starter compound. The alkoxylation is conducted by combining the cannabinoid, the DMC catalyst and alkylene oxide. The DMC catalyst is then allowed to become activated in the presence of the alkylene oxide. Once the DMC catalyst has become activated, the mixture is subjected to conditions sufficient to polymerize the alkylene oxide. In this manner, the cannabinoid becomes alkoxylated until poly(oxyalkylene) chains of a desired length are introduced. For instance, if the DMC catalyst and the cannabinoid (e.g., CBD), are mixed, and introduced to EO, ethoxylated-CBD (e-CBD) is formed.

In order to activate the DMC catalyst in the presence of the cannabinoid and alkylene oxide, several reaction conditions should be met, including (i) appropriate selection of the catalyst concentration, (ii) optimal reaction temperature and (iii) moisture content in the cannabinoid. To activate the catalyst with the cannabinoid, in one embodiment the (i) loading of the DMC catalyst may be in the range of about 1-1000 ppm, more preferably about 20-350 ppm. In another embodiment the (ii) reaction temperature may be in the range of about 110°-160°C, more preferably about 120°-140°C. Lastly, in one embodiment the (iii) water content in the cannabinoid, for example CBD, may be below about 1000 ppm, yet more preferably below about 400 ppm.

The alkoxylation may be performed by first mixing the cannabinoid, the DMC catalyst and the alkylene oxide and allowing the mixture to sit for a period of time at room temperature or an elevated temperature. When these materials are mixed, a so-called induction period occurs, during which the oxyalkylene reaction occurs very slowly. The induction period may range from a few minutes to several hours, depending on the particular DMC catalyst that is used and the temperature. During this induction period, the DMC catalyst becomes activated, and rapid polymerization of the alkylene oxide then commences.

In one embodiment, the starting mixture of DMC catalyst, cannabinoid and alkylene oxide is conveniently made by combining the DMC catalyst and cannabinoid in a pressure reactor (or by forming the catalyst in the reactor), and then pressurizing the reactor with an initial quantity of alkylene oxide. The induction period follows, as indicated by a nearly constant or slowly decreasing pressure in the reactor. The onset of rapid polymerization that follows the induction period is evidenced by a drop in pressure as the alkylene oxide is consumed.

The starting mixture of DMC catalyst, cannabinoid and alkylene oxide may be brought to any convenient temperature to activate the catalyst, such as from abort 110°C to about 160°C which is also suitable for conducting the polymerization once the DMC catalyst is activated.

Depending on the desired degree of alkoxylation, all the necessary alkylene oxide may be added to the reactor at the outset. It is usually preferred to add more alkylene oxide to the reactor once the DMC catalyst has become activated, especially when making higher molecular weight alkoxylated cannabinoids. A convenient way of adding the alkylene oxide is to pressurize the reactor with alkylene oxide and allow alkylene oxide to feed to the reactor on demand, maintaining a more or less constant pressure inside the reactor. Alternatively, any additional alkylene oxide may be fed in one or more discrete increments.

The total amount of alkylene oxide that is fed will depend on the desired equivalent weight of the product. The present disclosure is particularly suited for polymerizing at least about 1 mole of alkylene oxide per equivalent of cannabinoid compound. Similarly, the selection of the alkylene oxide will depend to a large extent on the intended end-use of the alkoxylated product. In one embodiment, the alkylene oxides polymerized with the cannabinoid/DMC catalyst are ethylene oxide, propylene oxide, 1,2-butylene oxide, and styrene oxide. Mixtures of these alkylene oxides can be used, and two or more of them can be polymerized sequentially to make block copolymers.

The polymerization reaction may be performed continuously or batchwise. In such continuous processes, the cannabinoid/DMC catalyst mixture is continuously fed into a continuous reactor such as a continuously stirred tank reactor (CSTR) or a tubular reactor. A feed of alkylene oxide is introduced into the reactor and the alkoxylated product continuously removed.

In one embodiment, the DMC catalyst may be supported. One method of making a supported DMC catalyst is by precipitating the catalyst in the presence of a polycarboxyl or polycarboxylate compound, as described in WO 01/04180, the contents of which is incorporated herein by reference. Supported DMC catalysts are also described in WO 99/44379 and U.S. Pat. No. 6,348,565, the contents of which are incorporated herein by reference.

The alkoxylated product that results from the above reaction conditions can be characterized using common analytical techniques, including hydroxyl value, melting point, unreacted cannabinoid (wt%), cloud point (5 wt% in water), pH (5 wt% in water), ¹H NMR-structural analysis, and appearance. Comparisons between a cannabinoid and its alkoxylated form (such as CBD and e-CBD in EXAMPLE 1 below) are made in Table 1 exemplifying how the e-CBD having a (POE) value of 12 behaves differently as compared to its non-ethoxylated precursor. While these properties are strongly dependent on the number of moles of EO used in relation to the equivalents of CBD, in order to obtain a water soluble or dispersible product, the number of moles of EO in the e-CBD may be between about 1-100, more preferably between about 10-25.

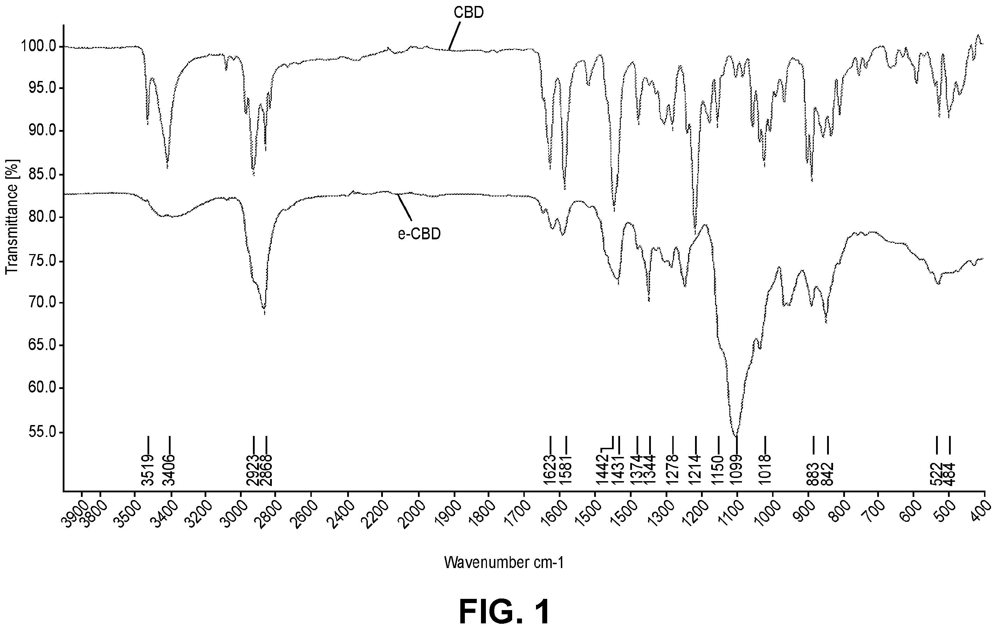

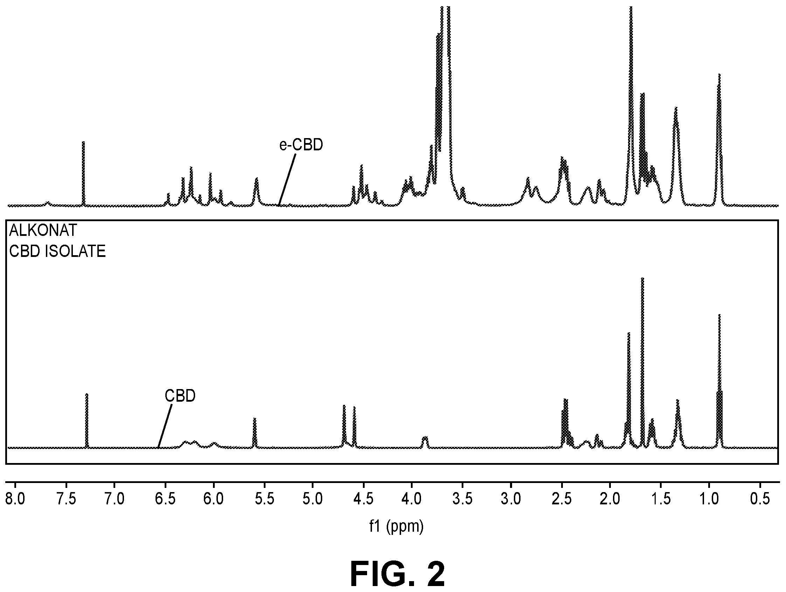

The use of DMC as a catalyst for the alkoxylation of a cannabinoid, most notably the ethoxylation of CBD using the DMC catalyst, was shown to maintain the structure composition of the CBD molecule, as evident through the FTIR and ¹H NMR spectra of CBD and e-CBD (see Figure 1 and Figure 2 respectively). This is a surprising observation, as other common alkoxylation methodologies have been demonstrated to alter this compound's molecular structure.

By varying the molar ratio of the epoxide-to-cannabinoid starter compound to produce a product having a (POE) value between 7 and 36, the hydrophilicity of the cannabinoid may be tuned (see Table 2). The water solubility of the alkoxylated-CBD compounds (Examples 1-4) was determined at room (25°C) and physiological (37°C) temperatures.

**Table 1. Physical Properties of CBD and Example 1**

| **Analytical Method** | **CBD** | **EXAMPLE 1** | |
|---|---|---|---|
| Appearance @25°C | White Powder | Yellow-to-Orange Paste | |
| Free CBD Alcohol (wt%) | ≥ 98.0 | | 5.2 |
| pH (5 wt% in water at 25°C) | Insoluble | | 6.76 |
| Melting Point (°C) | 65 | | 40-50 |
| | | See | Figure 1 |
| IR-Spectra | Pass | | |
| | | See | Figure 2 |
| ¹H NMR | Pass | | |
| | | See | Figure 3 |
| GC-FID | Pass | | |
| Hydroxyl-Value (mgKOH/g) | 178^{a} | | 70.7 |
| Water Solubility (5 wt% in water at 37°C) | Insoluble | | Soluble |

| | | | |
|---|---|---|---|
| a-Theoretical value based on the MW of CBD (315 g/mol). | | | |

**Table 2. Water Solubility of e-CBD Compounds Prepared using DMC.**

| Example | POE | Water Solubility (5wt% actives in water at 25°C | Water Solubility (5wt% actives in water at 37°C | Cloud Point (°C, Swt% actives in water) |
|---|---|---|---|---|
| CBD | 0 | Insoluble | Insoluble | Insoluble |
| Example 1* | 12 | Dispersible | Soluble | 60°C |
| Example 2 | 7 | Insoluble | Insoluble | Insoluble |
| Example 3 | 24 | Soluble | Soluble | 83°C |
| Example 4 | 36 | Soluble | Soluble | 97°C |

| | | | | |
|---|---|---|---|---|
| *Example 1 exhibits 2-step phase characteristics, in which two clouds points are observed at a loading of 5 wt% in water, (i) a faint phase change from slight cloudy-to-clear at 30°C, and (ii) a dramatic phase change from a clear solution-to-a milky emulsion at 60°C. No other e-CBD exhibits this behavior. | | | | |

### EXAMPLE 1

The DMC catalyst, product name "ARCOL CAT. 3 DRY", commercially available from Covestro (1 part) was dispersed in a small amount of melted cannabidiol (5-10 wt% of the total amount of CBD), which was then added to an Anton Parr reactor that contained the remaining cannabidiol (1132 parts in total). The solution was heated to 110°C and a vacuum was applied for one hour to remove the excess water from the solution. Once dewatered, a nitrogen header was applied, and the temperature was raised to 140°C while stirring. At this temperature, ethylene oxide (207 parts) was added. After a sudden drop in the reactor pressure was observed, the remainder of the ethylene oxide (EO) was added via continuous addition until all EO (1695 parts) was rapidly consumed. After which, the sample was cooled to 110°C, where it was deodorized via vacuum stripping and nitrogen purge. Typical characterization of the ethoxylate having a (POE) value of 12 included determination of the hydroxyl-value (OH-value), the amount of free-alcohol present in the final product and the water solubility was determined (see Table 1).

### EXAMPLE 2

The DMC catalyst, Product name "ARCOL CAT. 3 DRY", commercially available from Covestro (1 part) was dispersed in a small amount of melted cannabidiol (5-10 wt% of the total amount of CBD), which was then added to an Anton Parr reactor that contained the remaining cannabidiol (1333 parts in total). The solution was heated to 110°C and a vacuum was applied for one hour to remove the excess water from the solution. Once dewatered, a nitrogen header was applied, and the temperature was raised to 140°C while stirring. At this temperature, ethylene oxide (247 parts) was added. After a sudden drop in the reactor pressure was observed, the remainder of the ethylene oxide (EO) was added via continuous addition until all EO (1041 parts) was rapidly consumed. After which, the sample was cooled to 110°C, where it was deodorized via vacuum stripping and nitrogen purge. Characterization of the CBD-ethoxylate (POE-7) included the water solubility and cloud point (see Table 2).

### EXAMPLE 3

The DMC catalyst, Product name "ARCOL CAT. 3 DRY", commercially available from Covestro (1 part) was dispersed in a small amount of melted cannabidiol (5-10 wt% of the total amount of CBD), which was then added to an Anton Parr reactor that contained the remaining cannabidiol (1333 parts in total). The solution was heated to 110°C and a vacuum was applied for one hour to remove the excess water from the solution. Once dewatered, a nitrogen header was applied, and the temperature was raised to 140°C while stirring. At this temperature, ethylene oxide (558 parts) was added. After a sudden drop in the reactor pressure was observed, the remainder of the ethylene oxide (EO) was added via continuous addition until all EO (3925 parts) was rapidly consumed. After which, the sample was cooled to 110°C, where it was deodorized via vacuum stripping and nitrogen purge. Characterization of the CBD-ethoxylate (POE-24) included the water solubility and cloud point (see Table 2).

### EXAMPLE 4

The DMC catalyst, Product name "ARCOL CAT. 3 DRY", commercially available from Covestro (1 part) was dispersed in a small amount of melted cannabidiol (5-10 wt% of the total amount of CBD), which was then added to an Anton Parr reactor that contained the remaining cannabidiol (1333 parts in total). The solution was heated to 110°C and a vacuum was applied for one hour to remove the excess water from the solution. Once dewatered, a nitrogen header was applied, and the temperature was raised to 140°C while stirring. At this temperature, ethylene oxide (596 parts) was added. After a sudden drop in the reactor pressure was observed, the remainder of the ethylene oxide (EO) was added via continuous addition until all EO (6164 parts) was rapidly consumed. After which, the sample was cooled to 110°C, where it was deodorized via vacuum stripping and nitrogen purge. Characterization of the CBD-ethoxylate (POE-36) included the water solubility and cloud point (see Table 2).

## Claims

1. A process for preparing an alkoxylated cannabinoid comprising;
(i) forming a mixture of a cannabinoid, an alkylene oxide and a double metal cyanide (DMC) catalyst, the DMC catalyst being represented by the formula
M_{b}[M¹(CN)ᵣ(X)ₜ]_{c}[M²(X)₆]_{d} · nM³ₓA_{y}
where M and M³ are each metals; M¹ is a transition metal different from M, each X represents a group other than cyanide that coordinates with the M¹ ion; M² is a transition metal; A represents an anion; b, c and d are numbers that reflect an electrostatically neutral complex; r is from 4 to 6; t is from 0 to 2; x and y are integers that balance the charges in the metal salt M³ₓA_{y}, and n is zero or a positive integer, and
(ii) subjecting the mixture to conditions sufficient to activate the DMC catalyst and to alkoxylate the cannabinoid to form the alkoxylated cannabinoid.

2. The process according to claim 1, wherein the cannabinoid is an oily extract of a cannabis type plant, a pure cannabinoid, a synthetic cannabinoid or a cannabinoid derivative.

3. The process according to claim 2, wherein the cannabinoid is delta-9-tetrahydrocannabinol (THC), delta-8-tetrahydrocannabinol (THC), cannabidiol (CBD), cannabinodiol (CBND), cannabinol (CBN), cannabinol-C4 (CBN-C4), cannabigerol (CBG), cannabichromene (CBC), cannabicyclol (CBL), cannbivarol (CBV), tetrhydrocannabivarin (THCV), cannabidivarin (CBDV), cannabichromevarin (CBCV), cannbigerol monoethyl ether (CBGM), cannabielsoin (CBE), or cannabitriol (CBT).

4. The process according to claim 1, wherein the alkylene oxide is ethylene oxide (EO), propylene oxide (PO), butylene oxide (BO) or a mixture thereof.

5. The process according to claim 1, wherein the cannabinoid has a water content below about 1000 ppm.

6. The process according to claim 1, wherein the DMC catalyst is present in an amount in a range of about 1-1000 ppm.

7. The process according to claim 6, wherein the DMC catalyst is zinc hexacyanocobaltate.

8. The process according to claim 1, wherein the cannabinoid is cannabidiol, the alkylene oxide is ethylene oxide and the alkoxylated cannabinoid has a (POE) value of between 7 and 36.

9. The process according to claim 1, wherein the cannabinoid is alkoxylated at a temperature of in a range of about 110°-160°C.

10. A method for increasing the water solubility of a cannabinoid comprising alkoxylating the cannabinoid in the presence of an alkylene oxide and a DMC catalyst.

11. The method according to claim 10, wherein the water solubility of the cannabinoid after alkoxylating is increased by at least about 5% as compared to the water solubility of the cannabinoid before alkoxylating.

12. The method according to claim 11, wherein the water solubility of the cannabinoid after alkoxylating is increased by at least about 100% as compared to the water solubility of the cannabinoid before alkoxylating.

## Patentansprüche

1. Verfahren zur Herstellung eines alkoxylierten Cannabinoids umfassend:
(i) Bilden einer Mischung aus einem Cannabinoid, einem Alkylenoxid und einem Doppelmetallcyanid(DMC)-Katalysators, wobei der DMC-Katalysator dargestellt ist durch die Formel
M_{b}[M¹(CN)ᵣ(X)ₜ]_{c}[M²(X)₆]_{d} · nM³ₓA_{y},
wobei M und M³ jeweils Metalle sind; M¹ ein von M verschiedenes Übergangsmetall ist, jedes X eine von Cyanid verschiedene Gruppe darstellt, die mit dem M¹⁻lon koordiniert, M² ein Übergangsmetall ist; A ein Anion darstellt; b, c und d Zahlen sind, die einen elektrostatisch neutralen Komplex widerspiegeln; r von 4 bis 6 ist; t von 0 bis 2 ist; x und y ganze Zahlen sind, die die Ladungen im Metallsalz M³ₓA_{y}, ausgleichen, und n Null oder eine positive ganze Zahl ist, und
(ii) Unterwerfen der Mischung unter Bedingungen, die ausreichend sind, um den DMC-Katalysator zu aktivieren und das Cannabinoid zu alkoxylieren, um das alkoxylierte Cannabinoid zu bilden.

2. Verfahren nach Anspruch 1, wobei das Cannabinoid ein Ölextrakt einer Pflanze vom Cannabistyp, ein reines Cannabinoid, ein synthetisches Cannabinoid oder ein Cannabinoid-Derivat ist.

3. Verfahren nach Anspruch 2, wobei das Cannabinoid delta-9-Tetrahydrocannabinol (THC), delta-8-Tetrahydrocannabinol (THC), Cannabidiol (CBD), Cannabinodiol (CBND), Cannabinol (CBN), Cannabinol-C4 (CBN-C4), Cannabigerol (CBG), Cannabichromen (CBC), Cannabicyclol (CBL), Cannabivarol (CBV), Tetrahydrocannabivarin (THCV), Cannabidivarin (CBDV), Cannabichromevarin (CBCV), Cannabigerolmonoethylether (CBGM), Cannabielsoin (CBE) oder Cannabitriol (CBT) ist.

4. Verfahren nach Anspruch 1, wobei das Alkylenoxid Ethylenoxid (EO), Propylenoxid (PO), Butylenoxid (BO) oder eine Mischung davon ist.

5. Verfahren nach Anspruch 1, wobei das Cannabinoid einen Wassergehalt unterhalb von etwa 1000 ppm aufweist.

6. Verfahren nach Anspruch 1, wobei der DMC-Katalysator in einer Menge in einem Bereich von etwa 1 - 1000 ppm vorliegt.

7. Verfahren nach Anspruch 6, wobei der DMC-Katalysator Zinkhexacyanocobaltat ist.

8. Verfahren nach Anspruch 1, wobei das Cannabinoid Cannabidiol ist, das Alkylenoxid Ethylenoxid ist und das alkoxylierte Cannabinoid einen (POE)-Wert von zwischen 7 und 36 aufweist.

9. Verfahren nach Anspruch 1, wobei das Cannabinoid bei einer Temperatur in einem Bereich von etwa 110°-160°C alkoxyliert wird.

10. Verfahren zur Erhöhung der Wasserlöslichkeit eines Cannabinoids umfassend das Alkoxylieren des Cannabinoids in Gegenwart eines Alkylenoxids und eines DMC-Katalysators.

11. Verfahren nach Anspruch 10, wobei die Wasserlöslichkeit des Cannabinoids nach dem Alkoxylieren um wenigstens etwa 5 % erhöht ist, verglichen mit der Wasserlöslichkeit des Cannabinoids vor dem Alkoxylieren.

12. Verfahren nach Anspruch 11, wobei die Wasserlöslichkeit des Cannabinoids nach dem Alkoxylieren um wenigstens etwa 100 % erhöht ist, verglichen mit der Wasserlöslichkeit des Cannabinoids vor dem Alkoxylieren.

## Revendications

1. Procédé de préparation d'un cannabinoïde alkoxylé comprenant :
(i) la formation d'un mélange comprenant un cannabinoïde, un oxyde d'alcylène et un catalyseur au cyanure double métallique (DMC), le catalyseur DMC étant représenté par la formule
M_{b}[M¹(CN)ᵣ(X)ₜ]_{c}[M²(X)₆]_{d} · nM³ₓA_{y}
où M et M³ sont chacun des métaux ; M¹ est un métal de transition différent de M, chaque X représente un groupe autre que le cyanure qui se coordonne avec l'ion M¹ ; M² est un métal de transition ; A représente un anion ; b, c et d sont des nombres qui reflètent un complexe électrostatiquement neutre ; r va de 4 à 6 ; t va de 0 à 2 ; x et y sont des entiers qui équilibrent les charges dans le sel métallique M³ₓAᵥ, et n est zéro ou un entier positif, et
(ii) la soumission du mélange à des conditions suffisantes pour activer le catalyseur DMC et pour alkoxyler le cannabinoïde afin de former le cannabinoïde alkoxylé.

2. Procédé selon la revendication 1, dans lequel le cannabinoïde est un extrait huileux d'une plante de type cannabis, un cannabinoïde pur, un cannabinoïde synthétique ou un dérivé de cannabinoïde.

3. Procédé selon la revendication 2, dans lequel le cannabinoïde est le delta-9-tétrahydrocannabinol (THC), le delta-8-tétrahydrocannabinol (THC), le cannabidiol (CBD), le cannabinodiol (CBND), le cannabinol (CBN), le cannabinol-C4 (CBN-C4), le cannabigérol (CBG), le cannabichromène (CBC), le cannabicyclol (CBL), le cannabivarol (CBV), la tétrahydrocannabivarine (THCV), la cannabidivarine (CBDV), la cannabichromevarine (CBCV), l'éther monéthylique de cannabigérol (CBGM), le cannabielsoïne (CBE), ou le cannabitriol (CBT).

4. Procédé selon la revendication 1, dans lequel l'oxyde d'alcylène est l'oxyde d'éthylène (EO), l'oxyde de propylène (PO), l'oxyde de butylène (BO) ou un mélange de ceux-ci.

5. Procédé selon la revendication 1, dans lequel le cannabinoïde a une teneur en eau inférieure à environ 1000 ppm.

6. Procédé selon la revendication 1, dans lequel le catalyseur DMC est présent dans une quantité comprise dans une plage d'environ 1 à 1000 ppm.

7. Procédé selon la revendication 6, dans lequel le catalyseur DMC est l'hexacyanocobaltate de zinc.

8. Procédé selon la revendication 1, dans lequel le cannabinoïde est le cannabidiol, l'oxyde d'alcylène est l'oxyde d'éthylène et le cannabinoïde alkoxylé a une valeur (POE) comprise entre 7 et 36.

9. Procédé selon la revendication 1, dans lequel le cannabinoïde est alkoxylé à une température comprise dans une plage d'environ 110° à 160°C.

10. Méthode pour augmenter la solubilité dans l'eau d'un cannabinoïde comprenant l'alkoxylation du cannabinoïde en présence d'un oxyde d'alcylène et d'un catalyseur DMC.

11. Méthode selon la revendication 10, dans laquelle la solubilité dans l'eau du cannabinoïde après alkoxylation est augmentée d'au moins environ 5 % par rapport à la solubilité dans l'eau du cannabinoïde avant alkoxylation.

12. Méthode selon la revendication 11, dans laquelle la solubilité dans l'eau du cannabinoïde après alkoxylation est augmentée d'au moins environ 100 % par rapport à la solubilité dans l'eau du cannabinoïde avant alkoxylation.
